# EUROPEAN PATENT APPLICATION

(11) **EP 2 022 531 A1**
(43) Date of publication of application: **11.02.2009**
(21) Application number: 08013428.1
(22) Date of filing: 25.07.2008
(51) Int. Cl.: A61M 39/04, A61M 39/22, A61M 39/28

(54) **Liquid coinjector**

(30) Priority: 31.07.2007 JP 2007198739
(71) Applicant: Covidien AG, 8212 Neuhausen am Rheinfall (CH)
(72) Inventor: Kitani, Ichiro, Shizuoka-ken (JP); Funamura, Shigeaki, Shizuoka-ken (JP)
(74) Representative: Olsson, Carl H.S.

(57) **Abstract**

A liquid coinjector (A) is disclosed which includes a main body (10) having an upstream pipe (13) defining an inlet flow channel in fluid communication with a chamber portion (11) of the body, a downstream pipe (14) defining an outlet flow channel in fluid communication with the chamber portion, and a merging pipe (12) in fluid communication with the chamber portion. A flexible tube (24) is supported in the inlet flow channel of the upstream pipe. The flexible tube facilitates fluid flow from the upstream pipe to the chamber portion. At least one pressing part (23) is positioned adjacent to the flexible tube. The at least one pressing part is movable from a first position to a second position to compress the flexible tube and close the inlet flow channel.

## Description

### Background

### 1. Technical Field:

The present invention pertains to a type of liquid coinjector, which has a flow channel for feeding a liquid from an upstream pipe via a chamber portion to a downstream pipe, and a flow channel for feeding another liquid from a merging pipe via the chamber portion to the downstream pipe.

### 2. Background of Related Art

In the prior art, a transfusion tube is used to feed a prescribed physiological saline solution, liquid drug, or another liquid into the body of a patient. In such case, a dosage adjusting device may be used to adjust the flow rate of the liquid flowing in the transfusion tube. This dosage adjusting device includes an inner wall with a lower portion formed into a valve chamber, a main body composed of two tube joints having through-holes extending from two sides of the valve chamber outward, respectively, and a rotatable movable portion set in the inner wall of the main body. The two tube joints of the main body are formed such that their central axes deviate from each other in a vertical direction, and the lower portion of the movable portion is formed on a slope. Consequently, by rotating the movable portion, it is possible to open/close the through-holes while the openness of the through-holes of the two tube joints is changed. As a result, the flow rate of the liquid fed from the tube joint on one side via the valve chamber to the tube joint on the other side can be adjusted as desired.

The known dosage adjusting device described above is used for a transfusion tube for feeding one type of liquid into the body of the patient, and it cannot be used for a transfusion tube for feeding plural types of liquids into the body. Consequently, a type of liquid coinjector has been developed that can feed plural types of liquids to the patient by selectively turning ON/OFF various plural transfusion tubes. For a liquid coinjector for feeding plural liquids, when one liquid is fed, if another liquid is also simultaneously fed, backflow of the other liquid occurs and it can invade the upstream side of the tube for feeding the one liquid. In order to prevent backflow of liquid, a liquid coinjector has been developed that can feed any liquid by rotating a valve to switch on the flow path. In a conventional liquid coinjector, since the valve is rotated to turn ON/OFF any tube among plural tubes, the operation of switching of the flow path is complicated.

The objective of the present disclosure is to solve the aforementioned problems of the prior art by providing a scheme that can prevent backflow of a liquid drug or another liquid, and at the same time, can facilitate the operation for turning ON/OFF any tube among plural tubes.

### SUMMARY

A liquid coinjector is disclosed which includes a main body having an upstream pipe defining an inlet flow channel in fluid communication with a chamber portion of the body, a downstream pipe defining an outlet flow channel in fluid communication with the chamber portion, and a merging pipe in fluid communication with the chamber portion. A flexible tube is supported in the inlet flow channel of the upstream pipe. The flexible tube facilitates fluid flow from the upstream pipe to the chamber portion. At least one pressing part is positioned adjacent to the flexible tube. The at least one pressing part is movable from a first position to a second position to compress the flexible tube and close the flow channel.

In one embodiment, the at least one pressing part includes two pressing parts and the flexible tube is supported between the two pressing parts. Each pressing part can be supported on a first end of a rocking piece. The rocking piece can have a second end supported on an outer side of the main body. The second end of each pressing part can be pivotally supported on the main body.

In one embodiment, an upstream end of the flexible tube is fixed to an inner peripheral surface of the upstream pipe via a cylindrical fixing member. The flexible tube is coaxially supported within the upstream pipe

The merging pipe can include an open inlet end supporting a resilient sealing member positioned to seal the open inlet end. The sealing member can include a slit which extends through the sealing member.

The flexible tube can be formed from a resilient material which urges the at least one pressing part to the first position.

In one embodiment, the rocking piece includes a plate-shaped rib for stabilizing the rocking piece. The plate-shaped rib can include an engagement part and the main body can include an engaged part, the engagement part being configured to engage the engaged part in the first position to prevent outward movement of the rocking piece.

### BRIEF DESCRIPTION OF THE DRAWINGS

Various embodiments of the presently disclosed liquid coinjector are disclosed herein with reference to the drawings, wherein:
FIG. 1 is a perspective view of one embodiment of the presently disclosed liquid coinjector with the rocking pieces uncompressed;
FIG. 2 is a plan view of the liquid coinjector as shown in FIG. 1;
FIG. 3 is a front view of the liquid coinjector as shown in FIG. 1;
FIG. 4 is a left side view of the liquid coinjector as shown in FIG. 1;
FIG. 5 is a right side view of the liquid coinjector as shown in FIG. 1;
FIG. 6 is a cross-sectional view taken across section lines 6-6 in FIG. 2;
FIG. 7 is a cross-sectional view taken across section lines 7-7 in FIG. 3;
FIG. 8 is a cross-sectional view taken across section lines 8-8 in FIG 3;
FIG. 9 is a perspective view illustrating the liquid coinjector shown in FIG. 1 while the rocking pieces are pressed;
FIG.10 is a cross-sectional view illustrating the state of the liquid coinjector shown in FIG. 9 as seen from the front;
FIG. 11 is a cross-sectional view illustrating the state of the liquid coinjector shown in FIG. 9 as seen from the top; and
FIG.12 is a cross-sectional view illustrating the state of the liquid coinjector shown in FIG. 9 as seen from a lateral side.

### DETAILED DESCRIPTION OF EMBODIMENTS

Embodiments of the presently disclosed liquid coinjector will now be described in detail with reference to the drawings wherein like reference numerals designate identical or corresponding elements in each of the several views.

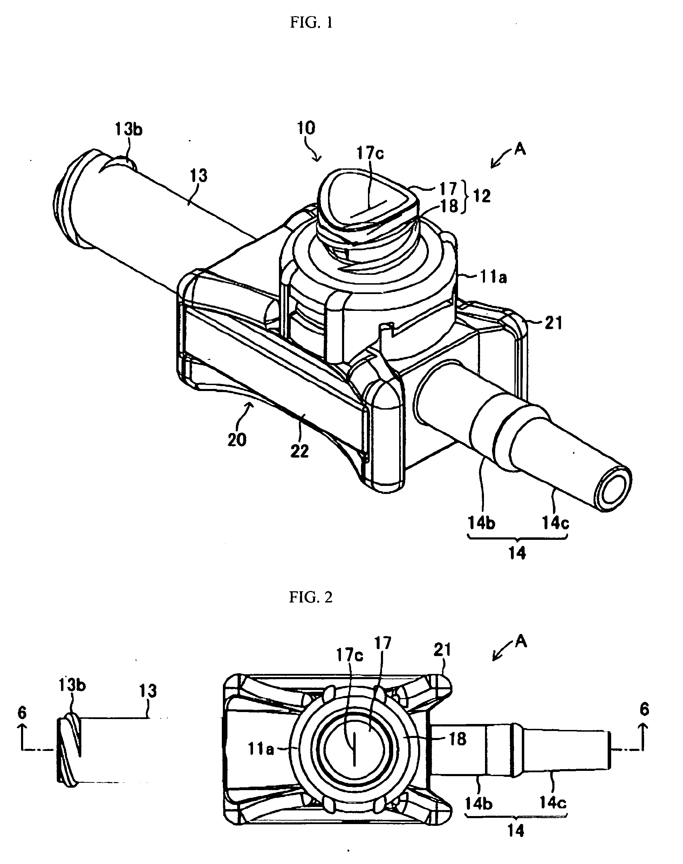

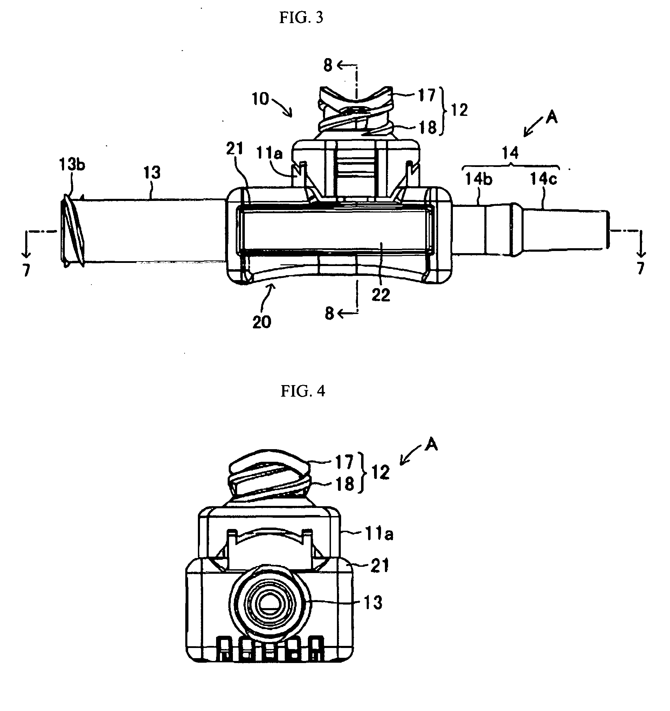

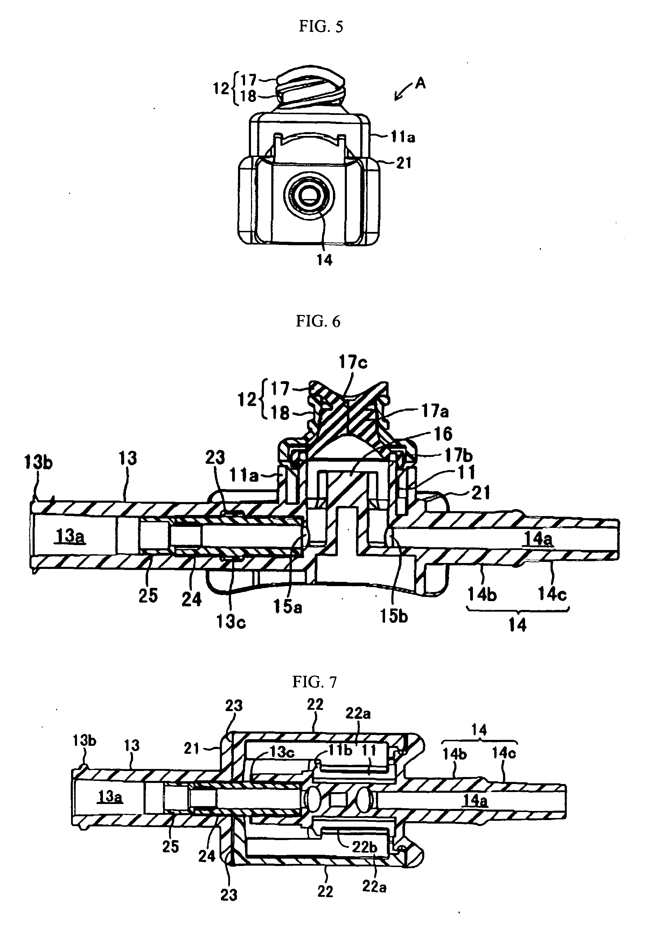

In the following, an explanation will be given in more detail regarding the liquid coinjector in an embodiment of the present disclosure. Figures 1-5 illustrate liquid coinjector A pertaining to one presently disclosed embodiment. This liquid coinjector A includes a liquid coinjector main body (10) and ON/OFF operation part (20). The liquid coinjector main body (10) includes a chamber portion (11) having a substantially cylindrical shape with a small length in the axial direction. The axial direction of the chamber portion (11) is vertically oriented (see Figures 6-8). Merging pipe (12) is set above chamber portion (11), and upstream pipe (13) which defines an inlet flow channel and downstream pipe (14) which defines an outlet flow channel extend from the left/right sides of chamber portion (11), respectively, and are coaxial with each other.

The chamber portion (11) is formed as a bottomed cylindrical member having a closed lower end and an open upper end. A frame-shaped attachment part (21) is formed on the outer periphery of chamber portion (11). Attachment part 21 has a substantially square shape in plan view and has opening portions set on the front side and rear side. Also, as shown in Figures 6 and 7, connecting holes (15a), (15b) are formed on the left/right opposite portions on the lower side from the center in the axial direction of chamber portion (11). Upstream pipe (13) is set in the portion of chamber portion (11) corresponding to connecting hole (15a). Flow channel (13a) is formed inside upstream pipe (13) and is connected to the interior of chamber portion (11) via connecting hole (15a). Downstream pipe (14) is set in the portion of chamber portion (11) corresponding to connecting hole (15b). The interior of chamber portion (11) and flow channel (14a), which is formed inside downstream pipe (14), are connected to each other via connecting hole (15b).

In one embodiment, chamber portion (11), upstream pipe (13), downstream pipe (14) and attachment part (21) are formed monolithically. Alternatively, one or more of the components can be separately formed and fastened together using known techniques. Also, the opening side portion of flow channel (13a) formed inside upstream pipe (13) has a tapered shape with a smaller diameter on the side of chamber portion (11) which gradually increases towards the opening. Upstream pipe (13) is formed in a female luer shape. Threaded portion (13b) is formed on the outer periphery of the opening portion of upstream pipe (13). The downstream pipe (14) includes a base end portion (14b) positioned on the side of chamber portion (11) and a smaller diameter male luer portion (14c) positioned on a tip side of downstream pipe (14). Male luer portion (14c) has a tapered shape with a diameter that is gradually reduced from the side of base end portion (14b) towards the tip side.

Cylindrical engaged portion (11a) is formed on the periphery of a cylindrical opening or inlet positioned in the upper portion of chamber portion (11). A space is formed between engaged portion (11a) and the cylindrical opening portion of the upper portion of chamber portion (11). Vertical wall (16) is formed in chamber portion 11 and extends upward from nearly the center of chamber portion (11) (shown in Figures 6 and 7) from the internal bottom surface of chamber portion (11). This vertical wall (16) is set inside chamber portion (11) such that vertical wall (16) blocks the central portion of chamber portion (11) in the width direction orthogonal to the longitudinal axis of flow channel (13a) and flow channel (14a). The upper end portion of vertical wall (16) extends nearly to the upper end of chamber portion (11).

The merging pipe (12) includes a sealing member which can be formed of rubber cork (17), e.g., natural rubber or synthetic rubber, and is attached on the upper end portion of the cylindrical opening portion of chamber portion (11). A cap member (18) is attached to periphery of rubber cork (17) to secure rubber cork (17) on the upper end portion of the cylindrical opening portion of chamber portion (11). The rubber cork (17) includes a cork main body (17a) having a thick disk shape, and a nearly ribbon-shaped pair of fixing pieces (17b) extending from the two sides of cork main body (17a) on the lower end of cork main body (17a). The two fixing pieces (17b) are positioned on the outer periphery of the upper end portion of the cylindrical opening portion of chamber portion (11), and cap member (18) is attached on its outer periphery.

The cap member (18) comprises a 2-step shaped cylindrical body having a larger diameter lower portion engaged with chamber portion (11) and a smaller diameter upper portion. Here, while fixing pieces (17b) are pressed on the cylindrical opening portion of chamber portion (11) by the upper larger-diameter portion of cap member 18, the lower portion of the larger-diameter portion is inserted between the cylindrical opening portion of chamber portion (11) and engaged portion (11a). As a result, cap member (18) is fixed on chamber portion (11). Also, the smaller-diameter portion of the upper portion of cap member (18) is open. Cap member (18) has rubber cork or sealing member (17) fixed internally so as to prevent upward protrusion of the upper portion of rubber cork (17) and to prevent the entry of rubber cork (17) into cap member (18). A slit (17c) is formed in rubber cork (17) to provide a passage between the outer side and the interior of chamber portion (11).

This slit (17c) is normally closed by the elasticity of rubber cork (17). When the outer side and the interior of chamber portion (11) are connected to each other such as when a connector or the like (not shown in the figure) is inserted into slit (17c) of rubber cork (17), it is possible to form a flow channel into the connector or the like. For example, this connector may define a flow channel and include a male luer portion. When the male luer portion is inserted into slit (17c) of rubber cork (17), the flow channel of the connector and the interior of chamber portion (11) become fluidly coupled to each other. When this occurs, the male luer portion of the connector and the peripheral surface of slit (17c) are in close sealed contact with each other due to the elasticity of rubber cork (17).

The ON/OFF operation part (20) includes an attachment part (21) formed on the outer periphery of chamber portion (11), a pair of operation parts including a rocking piece (22) and a pressing part (23) connected to attachment part (21), a flexible tube (24) set inside upstream pipe (13), and a fixing member (25) which secures tube (24) in upstream pipe (13). In one embodiment, the attachment part (21) has a substantially square box shape body with a width that is a little larger than the height. On the front side and rear side of the square box shape body, rectangular openings which stretch in the left/right direction are formed, respectively. Also, a central portion of the bottom surface of the attachment part (21) is curved upward to form a curved surface, and define a hollow portion.

The hollow portion is formed continuously not only on the bottom surface portion of attachment part (21), but also on the lower portion of chamber portion (11). A portion of the hollow portion extends from the lower surface of vertical wall (16) to the inner side of chamber portion 11. By providing this hollow portion, when attachment part (21) is formed, it is possible to prevent generation of a depression, and it is possible to reduce the quantity of the molding material that is used. Also, the base end portions of rocking pieces (22) are connected to the edge portions on the side of downstream pipe (14) in two openings located in front of and behind attachment part (21), respectively. While two rocking pieces (22) have their base end portions connected to the opening edge portions, they can be rotatably attached between the opening peripheral edge portion of attachment part (21) and the inner side of attachment part (21) around the base end portions.

In one embodiment, each pressing part (23) is monolithically formed with rocking piece (22) at the tip portion of each rocking piece (22), and extends to the inner side of attachment part (21) orthogonal to rocking piece (22). Also, in upstream pipe (13), a through hole (13c) is formed through the outer peripheral surface to the inner peripheral surface of upstream pipe (13) in the portion facing the tips of the pressing parts (23). The tip portions of the pressing parts (23) are positioned inside through hole (13c). When rocking piece (22) is not pressed, the tip portion of each pressing part (23) is positioned on the inner peripheral surface of upstream pipe (13). On the other hand, when rocking piece (22) is pressed, the tip portion of each pressing part (23) enters the inner side of upstream pipe (13).

Reinforcing plate-shaped ribs (22a) are positioned on an inner surface of each rocking piece (22). Ribs (22a) extend from the base end side of rocking piece (22) to pressing part (23). A width of the plate-shaped ribs (22a) is larger on the base end side of rocking piece (22) than the width of the plate-shaped ribs (22a) on the side of pressing part (23), and a step portion is formed at a boundary portion. A small spacing is provided between the wide portion of plate-shaped ribs (22a) and the outer peripheral surface of chamber portion (11). A larger spacing is formed between the narrow portion of plate-shaped ribs (22a) and the outer peripheral surface of upstream pipe (13).

A hook-shaped engagement part (22b) is formed on the tip of the wide portion of plate-shaped rib (22a) of each rocking piece (22), and an engaged part (11b) is formed on the outer peripheral surface of chamber portion (11). Engagement between engagement part (22b) and engaged part (11b) prevents rocking piece (22) from protruding from attachment part (21). As a result, while rocking piece (22) can move or pivot into attachment part (21), it cannot protrude outwardly of attachment part (21). Also, the portion of chamber portion (11) facing the step portion of plate-shaped rib (22a) in engaged part (11b) is formed on a guide portion that is engaged in a slidable way with the step portion.

A tube (24) includes a cylindrical soft and flexible plastic body. The tube (24) is supported in close contact with the inner peripheral surface of upstream pipe (13) in the portion within flow channel (13a) from near the center of upstream pipe (13) to chamber portion (11). An upstream end of tube (24) is fixed by fixing member (25) on the inner peripheral surface of upstream pipe (13). The fixing member (25) is a 2-step shaped cylindrical body with a diameter of a base end side which is larger than that of the tip end side. The larger-diameter base end side is positioned on the upstream side, and its smaller-diameter tip side is positioned on the downstream side when fixing member (25) is supported in upstream pipe (13). That is, fixing member (25) is inserted into the upstream side portion of tube (24) such that the upstream end side portion of the tube (24) is held between the outer peripheral surface of the tip side portion and the inner peripheral surface of upstream pipe (13). The base end side portion of fixing member (25) is pressed in contact with the inner peripheral surface of upstream pipe (13) to secure fixing member (25) inside upstream pipe (13).

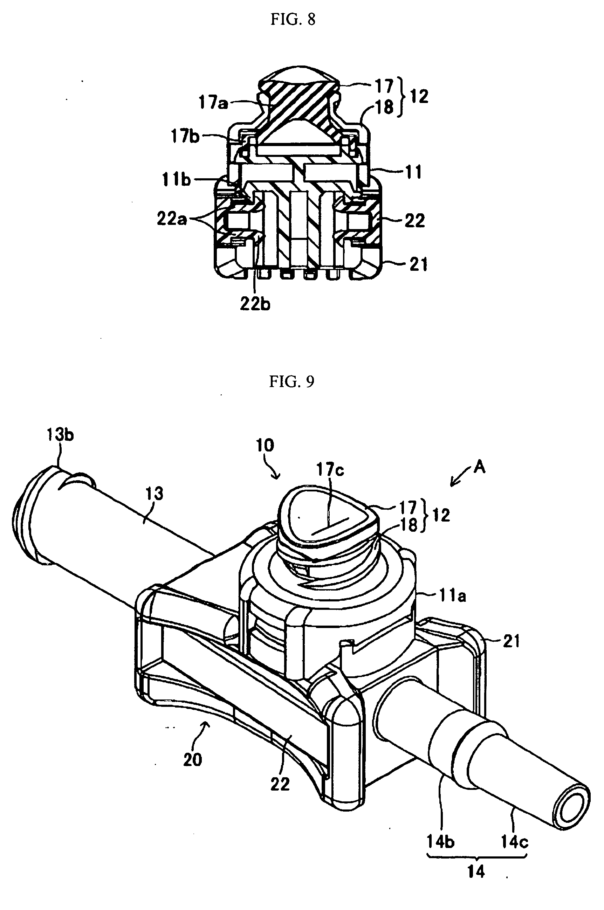

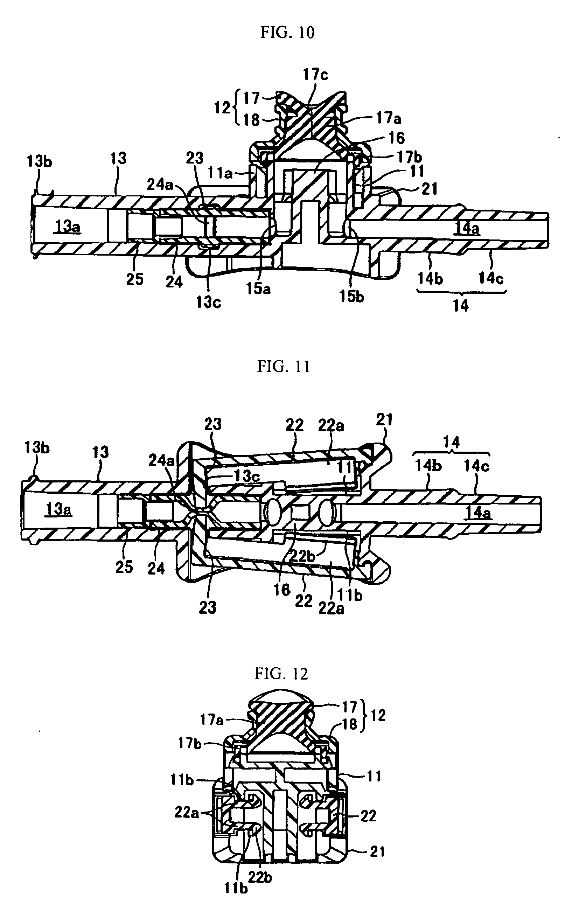

As shown in Figure 9, when the tip sides of rocking pieces (22) are pressed inwardly into attachment part (21), the tip side portions of the rocking pieces (22) and pressing parts (23) approach each other to obtain the state shown in Figures 10-12. As a result, portions (24a) of tube (24) are sandwiched between the pressing parts (23) and pressed into contact with each other to close the flow channel between flow channel (13a) of upstream pipe (13) and the interior of chamber portion (11). When the rocking pieces (22) are pressed inwardly, since the step portion of plate-shaped rib (22a) is guided by the guiding part in engaged part (11b) of chamber portion (11), rocking pieces (22) and pressing parts (23) move smoothly.

When the force for pressing the rocking pieces (22) into attachment part (21) is released, tube (24) recovers to its original cylindrical shape, and due to the recovering force of resilient tube (24), the tip side portions of rocking pieces (22) and the pressing parts (23) are urged outwardly from each other to return to the state shown in Figures 6-8. While tube (24) recovers to its original cylindrical shape, flow channel (13a) of upstream pipe (13) and the interior of chamber portion (11) are reconnected to each other. When a liquid drug or the like is fed from upstream pipe (13) to chamber portion (11), after the liquid drug or the like flows from flow channel (13a) into chamber portion (11) and rides over vertical wall (16), it flows from within chamber portion (11) into flow channel (14a) of downstream pipe (14). In this case, vertical wall (16) acts to prevent backflow of the liquid drug or the like, and, at the same time prevent stagnation of air inside chamber portion (11) when the liquid drug or the like passes through the chamber portion (11).

When a prescribed liquid drug is to be fed into the body of a patient (not shown in the figure), a female luer portion at the rear end portion of a transfusion tube (not shown in the figure), which is connected to a retention needle that is pierced into and retained on the patient, is connected to downstream pipe (14). Also, the male luer portion set on the tip portion of the transfusion tube extending from a container that contains the liquid drug to be fed to the patient is connected to upstream pipe (13). Then, while the retention needle is pierced into the body of the patient and retained there, the liquid drug in the container or the like is fed to the patient, so that liquid drug feeding is carried out. When another liquid drug in addition to the liquid drug fed from the container is to be fed to the patient, the other liquid drug is injected into merging pipe (12) and into chamber portion (11) via a transfusion tube connected to a connector.

In this case, when the connector is inserted into slit (17c) of rubber cork (17) in merging pipe (12), the connector and downstream pipe (14) are connected to each other via the flow path in chamber portion (11). Consequently, the liquid drug is injected from the connector side into the patient. When the tip end sides of rocking pieces (22) are pressed into the interior of attachment parts (21), tube (24) is closed as shown in Figures 10 and 11. Consequently, flow of the liquid drug fed from the container or the like via upstream pipe (13) into chamber portion (11) is stopped. In addition, backflow of the liquid drug injected from merging pipe (12) into chamber portion (11) to the side of upstream pipe (13) is prevented, so that the liquid can flow to downstream pipe (14) with high reliability.

When rocking pieces (22) are released after completion of injection of the liquid drug through merging pipe (12), portion (24a) of tube (24) that has been closed is opened, and the liquid drug can be fed again from upstream pipe (13) to the patient. Also, before the retention needle is pierced into the body of the patient and left there, a small quantity of the liquid drug or the like should be exhausted from the tip of the retention needle. In this way, air in the flow channel can be exhausted together with the liquid drug. Also, when the liquid drug that flows from the side of upstream pipe (13) through the interior of chamber portion (11) to the side of downstream pipe (14) passes through the interior of chamber portion (11), it rides over vertical wall (16) and passes through the upper side of the interior of chamber portion (11), so that it is possible to prevent stagnation of air inside chamber portion (11).

As discussed above, liquid coinjector A of the present embodiment includes a liquid coinjector main body (10) and an ON/OFF operation part (20). The liquid coinjector main body (10) includes a chamber portion (11), an upstream pipe (13), a downstream pipe (14) and a merging pipe (12) connected to the chamber portion (11). The operation part (20) includes a tube (24) supported in upstream pipe (13), pressing parts (23) and rocking pieces (22) for closing tube (24). Consequently, it is possible to feed a liquid drug or the like from upstream pipe (13) via chamber portion (11) to downstream pipe (14), and, at the same time, it is possible to feed another liquid drug from merging pipe (12) via chamber portion (11) to downstream pipe (14).

As rocking pieces (22) are pressed or undergo pressure release, it is possible to stop/reopen feeding of the liquid drug fed from upstream pipe (13) via chamber portion (11) to downstream pipe (14). Rubber cork (17) having slit (17c) and cap member (18) normally cuts merging pipe (12) off from chamber portion (11). However, when a needle or syringe or the like is pierced in slit (17c) of rubber cork (17), a flow channel between merging pipe (12) and chamber portion (11) is established to facilitate supply of a second type of liquid drug.

It is possible with liquid coinjector A to selectively establish a connection between upstream pipe (13) and chamber portion (11) by opening and closing tube (24). Also, it is possible to cut off or connect the flow channel from merging pipe (12) through chamber portion (11). When a second liquid drug is fed from the side of merging pipe (12) via chamber portion (11) to the side of downstream pipe (14) while tube (24) is closed with rocking pieces (22) pressed, the liquid drug does not flow back into the side of upstream pipe (13). Instead, it can flow to the side of downstream pipe (14) with high reliability. In this way, by means of liquid coinjector A pertaining to the present embodiment, it is possible to feed any liquid drug in an appropriate state into the body of a patient.

The operation for closing tube (24) of liquid coinjector A can be performed simply by holding and pressing the pair of rocking pieces (22) with two fingers of the operator. In addition, the operation for connecting the two ends of closed tube (24) can be performed by simply releasing the pressure applied on the pair of rocking pieces (22). Also, the operation for closing tube (24) is not performed by directly applying pressure on pressing parts (23). Instead, it is performed by manipulating rocking pieces (22) with a sufficiently large area for holding by the fingers. Consequently, the pressing operation can be performed even more simply. In addition, since tube (24) is fixed inside upstream pipe (13) by fixing member (25), even if tube (24) stretches under pressure of pressing parts (23), the state of fixing on the inner peripheral surface of upstream pipe (13) can still be maintained.

The presently disclosed liquid coinjector is not limited to the aforementioned embodiment. It is possible to make appropriate modifications to the embodiment. For example, in the embodiment, slit (17c) is formed on rubber cork (17), and a portion of a male luer is inserted in slit (17c) for connection of the connector to merging pipe (12). However, it is also possible to insert a male luer portion of a syringe or an injecting needle instead of a connector into the rubber cork. If an injecting needle is inserted, there is no need to form slit (17c) on the rubber cork (17). Also, in the embodiment, pressing parts (23) are manipulated by pressing rocking pieces (22) set at the tips of rocking pieces (22). However, one may also adopt a scheme in which rocking pieces (22) are omitted, and pressing parts (23) are directly pressed. As a result, attachment part (21) can be smaller. In addition, a variety of other changes are envisioned which are within the scope of the present disclosure.

## Claims

1. A liquid coinjector comprising:
a main body defining a chamber portion and including an upstream pipe defining an inlet flow channel in fluid communication with said chamber portion, a downstream pipe defining an outlet flow channel in fluid communication with said chamber portion, and a merging pipe in fluid communication with said chamber portion;
a flexible tube supported in said upstream pipe, said flexible tube facilitating fluid flow from said upstream pipe to said chamber portion;
and at least one pressing part positioned adjacent the flexible tube, the at least one pressing part being movable from a first position to a second position to compress the flexible tube and close the inlet flow channel.

2. The liquid coinjector according to Claim 1, wherein the at least one pressing part includes two pressing parts, said flexible tube being supported between the two pressing parts.

3. The liquid coinjector according to Claim 2, wherein each said pressing part is supported on a first end of a rocking piece, the rocking piece having a second end supported on an outer side of said main body.

4. The liquid coinjector according to Claim 1, wherein an upstream end of said flexible tube is fixed to an inner peripheral surface of said upstream pipe via a cylindrical fixing member, wherein said flexible tube is coaxial with said upstream pipe

5. The liquid coinjector according to Claim 1, wherein the merging pipe includes an open inlet end supporting a resilient sealing member positioned to seal the open inlet end.

6. The liquid coinjector according to Claim 5, wherein the sealing member includes a slit which extends through the sealing member.

7. The liquid coinjector according to Claim 2, wherein the second end of each pressing part is pivotally supported on the main body.

8. The liquid coinjector according to Claim 1, wherein the flexible tube is formed from a resilient material which urges the at least one pressing part to the first position.

9. The liquid coinjector according to Claim 3, wherein said rocking piece includes a plate-shaped rib for stabilizing said rocking piece.

10. The liquid coinjector according to Claim 9, wherein said plate-shaped rib includes an engagement part and said main body includes an engaged part, said engagement part being configured to engage the engaged part in the first position to prevent outward movement of said rocking piece.

11. The liquid coinjector according to Claim 6, wherein the slit is normally closed.
